# EUROPÄISCHE PATENTANMELDUNG

(11) **EP 1 306 790 A2**
(43) Veröffentlichungstag der Anmeldung: **02.05.2003**
(21) Anmeldenummer: 02020067.1
(22) Anmeldetag: 06.09.2002
(51) Int. Cl.: G06F 19/00

(54) **System zur Erstellung von Behandlungsplänen**

(30) Priorität: 26.09.2001 DE 10147470
(71) Anmelder: SIEMENS AKTIENGESELLSCHAFT, 80333 München (DE)
(72) Erfinder: Abraham-Fuchs, Klaus, 91058 Erlangen (DE); Rumpel, Eva, Dr., 91052 Erlangen (DE)

(57) **Zusammenfassung**

System zur Erstellung von Behandlungsplänen, insbesondere im Zuge von Disease management services zur Patientenaufklärung, -weiterbildung und -motivation, gegebenenfalls kombiniert mit Telemonitoring kritischer Körperwerte und daraus abgeleiteter frühzeitiger Erkennung von Risikosituationen mit einer Datenbank mit einer Vielzahl von in digitaler Form gespeicherten Behandlungsmodulen, die mithilfe von Operatoren miteinander zu einem Behandlungsplan verknüpfbar sind.

## Beschreibung

Die Erfindung bezieht sich auf ein System zur Erstellung von Behandlungsplänen, insbesondere im Zuge von Disease management services zur Patientenaufklärung, -weiterbildung und -motivation, gegebenenfalls kombiniert mit Telemonitoring kritischer Körperwerte und daraus abgeleiteter frühzeitiger Erkennung von Risikosituationen.

Bei diesen Disease management services wird mittelfristig auch eine intensive Nutzung des Internets angestrebt, wobei momentan die Patienten in der Regel per Post und - ganz zentral - per Telefon an ihren Disease management services provider (DMSP) angeschlossen sind. Die Ausdehnung dieses Services auf große Patientenzahlen bedarf jedoch einer weiteren Automatisierung und somit des Einsatzes von geeigneter Software. Diese Software muss jedoch nicht nur auf die im Fokus stehende Krankheit zugeschnitten sein, sondern auch auf jeden einzelnen Dienstleister, denn gerade im Wissen um Strukturen und Abläufe des jeweiligen Disease management services providers und im daraus entstehenden Betreuungskonzept liegen die Kernkompetenzen (und damit auch der Wettbewerbsvorteil) eines jeden dieser Unternehmer. Bisher werden diese Behandlungspläne von Hand erstellt, wobei sich die Branche mit allgemein gehaltener, nicht branchenspezifischer Software, wie zum Beispiel zur Unterstützung von Call-Centern behilft.

Das US-Patent 5,319,543 beschreibt einen Workflow Server für ein medizinisches Aufzeichnungssystem, bei dem es um eine Verbesserung des Workflow in der elektronischen Patientenakte und insbesondere um einen geregelten Zugriff für verschiedene Gruppen auf die Daten einer solchen elektronischen Patientenakte geht. Das System betrifft aber weder einen Disease Management Service noch geht es dabei um die Erstellung eines individuellen Behandlungsplanes für einen bestimmten Patienten.

In der DE 195 23 036 A1 ist ein automatischer Programmgenerator beschrieben, bei dem es um die Erstellung eines Anwenderprogramms für einen Computer und zwar speziell eines mit den Quellprogrammen arbeitenden Anwenderprogramms auf Basis des Maschinencodes geht. Die Erstellung eines solchen Anwenderprogramms auf Quellcodebasis soll dadurch erleichtert und weniger individuell von den Eigenheiten des jeweiligen Programmierers abhängen, dass Standartprogrammteile in halbfertiggestelltem Zustand zur Verfügung gestellt werden, die vom Nutzer zu einem fertigen Anwenderprogramm zusammengesetzt werden. Dieses unterstützte Erstellen von Anwenderprogrammen für Computer hat aber mit einem System zur Erstellung von medizinischen Behandlungsplänen nichts zu tun.

Der Erfindung liegt daher die Aufgabe zugrunde, ein System zur erleichterten Erstellung von Behandlungsplänen, insbesondere im Zuge von Disease management services zu schaffen.

Zur Lösung dieser Aufgabe ist ein derartiges System erfindungsgemäß gekennzeichnet durch eine Datenbank mit einer Vielzahl von in digitaler Form gespeicherten Behandlungsmodulen, die mithilfe von Operatoren miteinander zu einem Behandlungsplan verknüpfbar sind, wobei bevorzugt eine graphische Benutzeroberfläche, zur Unterstützung der Auswahl und der Zusammenstellung von vorgegebenen Behandlungsmodulen sowie Eingabeeinrichtungen für Patientendaten zur Individualisierung des Behandlungsplans vorgesehen sind.

In weiterer Ausgestaltung der Erfindung kann dabei vorgesehen sein, dass der Behandlungsplan in maschinenlesbarer Form ausgebildet ist und automatisch Aufgaben ausführt, wie Terminverwaltung, Protokollführung, Freischalten von Informationen aus dem Internet, Versenden von Emails, Konzipieren von Formularen für Datenbanken, Verwaltung für Patientendaten und vieles mehr. Neben der Automatisierung dieser Vorgänge erleichtert das in Software abgebildete Prozesswissen die innerbetriebliche Dokumentation der Vorgänge und bildet damit die Grundlage für ein Qualitätsmanagement. Darüber hinaus erleichtert das System den Transfer des Prozesswissens auf neue Mitarbeiter oder neue Geschäftsfelder.

Zur Erleichterung des Zusammenstellens der einzelnen Behandlungsmodule zu einem umfangreichen Behandlungsplan kann gemäß einem weiteren Merkmal der vorliegenden Erfindung vorgesehen sein, dass die Datenbank zusätzlich individuell abänderbare Standardbehandlungspläne aus jeweils einer Mehrzahl von Behandlungsmodulen für bestimmte Standardkrankheiten enthält. Auf diese Art und Weise lässt sich für viele in der Praxis ja auch häufig vorkommende Standardkrankheiten die Erstellung des Behandlungsplans wesentlich vereinfachen, da man nur einzelne Module zusätzlich auswählen oder in anderer Reihenfolge einbauen muss, um eine Anpassung für den jeweiligen Anwendungszweck oder einen ganz speziellen Patienten zu erreichen.

Die Prozessschritte des zu erstellenden Behandlungsplans werden aus dem vorgegebenen Pool möglicher Teilschritte der zeitlichen Reihenfolge der späteren Ausführung gemäß ausgewählt und zu einer zeitlich aneinandergereihten Modul-Kette angeordnet, wobei der Workflow sowohl linear sein kann, als auch als Entscheidungsbaum mit Entscheidungsknoten und/oder Schleifen ausgebildet sein kann.

Zum weitgehend intuitiven Arbeiten mit dem System können dabei Schaltflächen und Toolbox-Symbolleisten vorgesehen sein, wobei symbolhaft Standardelemente wie Dateneingang (aus Patientenstammdaten oder Patientenfragebogen), Schleifen (Beispiel Jahresabo: für i<12 versende monatlichen Rundbrief), Verzweigungen und Datenausgabe vorgesehen sein können.

Ganz allgemein ist die Anordnung entweder so getroffen, dass die gewünschten Module in einer textuellen Liste oder einer Anordnung graphischer Elemente (Ikons oder dergleichen) markiert und durch bestätigenden Mausklick oder Tastendruck in die Prozesskette übernommen werden, oder aber dass die gewünschten Module - insbesondere bei nichtlinearen Entscheidungsbäumen - durch "Drag & Drop", das heißt ziehen des markierten Moduls mit der Maus oder dergleichen an die entsprechende Stelle des Entscheidungsbaums eingebaut werden können.

Als Randwerte des Systems können das allgemeine medizinische Wissen (Leitlinien, Standards) und/oder unternehmenspezifisches Wissen (Prozesswissen, patienten- und/oder kostenträgerbezogenes zielgruppenspezifisches Wissen) mit eingehen.

Die Verwendung vordefinierter Makros einschließlich beispielsweise der Zusatzfunktion "copy-paste" zur zügigen Eingabe sich wiederholender Teilstrukturen, reduziert den individuellen Programmieraufwand und ermöglicht andererseits Standards (wie zum Beispiel die Therapieleitlinien der Fachverbände) in die unternehmenseigene Prozessführung aufzunehmen.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: einen Flussplan des erfindungsgemäßen Systems zur Erstellung eines individuellen Behandlungsplans aus vorgegebenen Modulen,
- Fig. 2: eine Darstellung der möglichen Automatisierung der Vorgänge innerhalb des individuellen Behandlungsplans und
- Fig. 3: eine detailliertere Darstellung des Systems zur Erstellung eines bestimmten Behandlungsplanes.

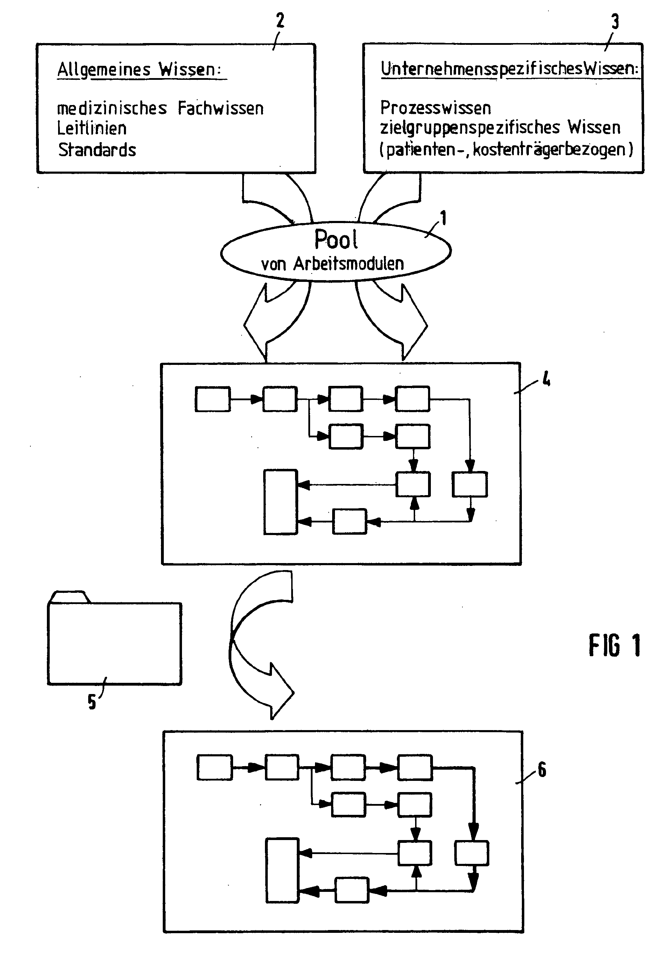

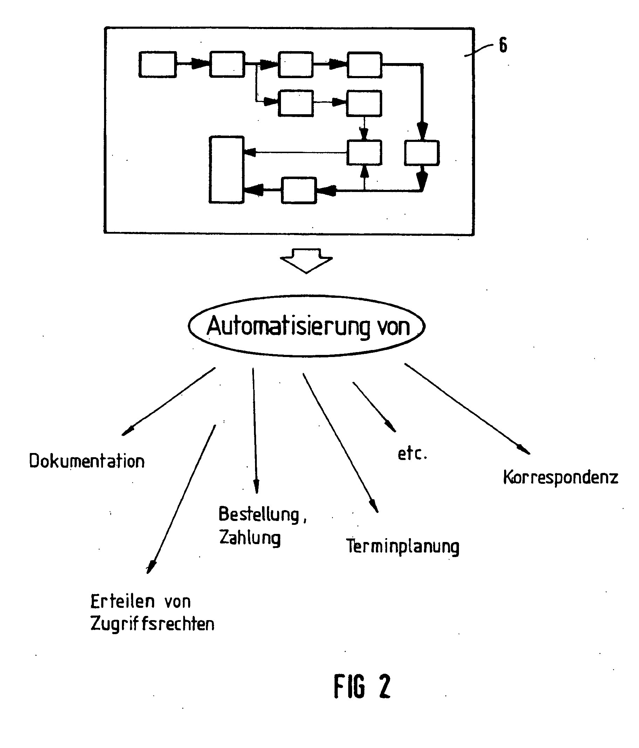

Aus einer Datenbank 1 von Behandlungsmodulen, der mit einer Datenbank 2 über das allgemeine medizinische Wissen sowie Leitlinien und Standards sowie einer Datenbank 3 über das unternehmensspezifische Wissen, wie Prozesswissen oder das patienten- und kostenträgerbezogene zielgruppenspezifische Wissen verbunden ist, kann über eine graphische Benutzeroberfläche eine allgemeine Prozessführung 4 erstellt werden, aus der unter Eingabe von Patientendaten 5 über eine entsprechende Eingabevorrichtung ein individueller Behandlungsplan 6 erstellt wird. Dieser individuelle Behandlungsplan 6 aus Fig. 1 ermöglicht - was in Fig. 2 dargestellt ist - die Automatisierung sowohl der Dokumentation als auch des Erteilens von Zugriffsrechten der Bestellung und Bezahlung, der Terminplanung, der Korrespondenz etc..

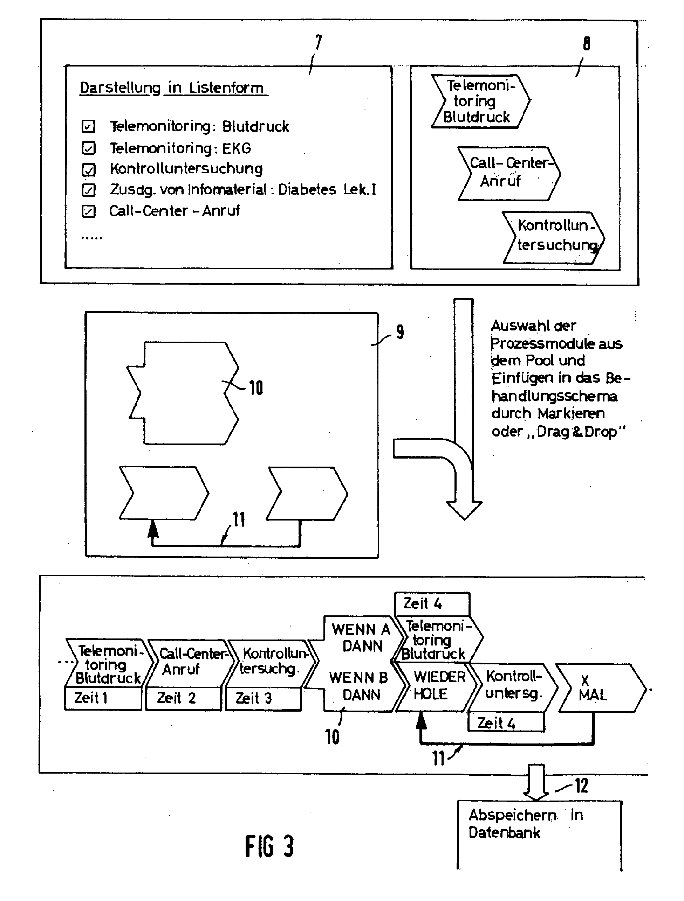

Innerhalb des Pools von Arbeitsmodulen sind einzelne in der allgemeinen Prozessführung 4 bzw. dem Behandlungsplan 6 als Kästchen wiedergegebene Module enthalten, die (vgl. insbes. Fig. 3) entweder in Form von Listen 7 oder als graphische Elemente 8 dargestellt sind und auf die die graphische Benutzeroberfläche Zugriff gestattet. Durch Markieren des Moduls in der textuellen Liste 7 oder in der Anordnung der graphischen Elemente 8 oder aber auch durch "Drag & Drop" lassen sich die jeweils markierten Module beispielsweise mit Hilfe eines Mausklicks oder Tastendrucks an die richtige Stelle im Entscheidungsbaum der allgemeinen Prozessführung 4 einbauen. Dabei enthält die Datenbank mit den Listen 7 oder den graphischen Elementen 8 natürlich auch noch eine Datenbank 9 mit Kontrollelementen, auf die ebenfalls die graphische Benutzeroberfläche Zugriff bietet, um entweder Entscheidungsknoten 10 oder Schleifen 11 zu bilden, die an entsprechender Stelle des Entscheidungsbaums eingebaut werden können.

Bei dem in Fig. 3 gezeigten sehr vereinfachten Ausführungsbeispiel ist beispielsweise zu einer Zeit 1 (beispielsweise täglich 20,00 Uhr) der Modul Tele-Monitoring Blutdruck aufzurufen, wobei zu einer Zeit 2, kurz nach der Zeit 1 oder auch in unregelmäßigen Abständen danach, ein Call-Center-Anruf erfolgen soll, der wiederum beispielsweise zu einer Zeit 3, also zum Beispiel jede erste Woche im Monat, eine Kontrolluntersuchung anregt. Über einen Entscheidungsknoten 10 kann zu einer Zeit 4 entweder ein erneutes Tele-Monitoring Blutdruck gefordert werden oder aber eine Wiederholung einer Kontrolluntersuchung, wobei schließlich über eine Schleife 11 diese Kontrolluntersuchung beliebig oft vorgebbar wiederholt werden kann.-Schließlich ist das Abspeichern des Behandlungsplans und seiner Ergebnisse in einer Speicherdatenbank 12 angedeutet.

Das in Fig. 3 unten gezeigte individuelle Behandlungsschema für einen Patienten kann entweder in der Weise realisiert sein, dass der Arztcomputer die Behandlungspläne der Patienten abfragt oder aber dass der Behandlungsplan für den einzelnen Patienten sich von sich aus meldet und beispielsweise mit dem Arztcomputer eine "Terminabsprache trifft".

Das erfindungsgemäße Verfahren und die dazugehörigen Software-Tools unterstützen einen medizinischen Leistungserbringer darin, auf eine sehr zeiteffiziente Art und Weise einen individuellen Behandlungsplan für einen Patienten zusammenzustellen und mit Hilfe des im Computer gespeicherte Behandlungsplans dessen Durchführung zu überwachen bzw. teilautomatisch auszuführen. Der Behandlungsplan kann dabei auch Aktivitäten und Entscheidungen aller am Prozess Beteiligten beinhalten, zum Beispiel des Arztes, des Patienten, des betreuenden Pflegepersonals eines Call-Centers oder der Sprechstundenhilfe.

Die Erfindung ist nicht auf die dargestellten Ausführungsbeispiele beschränkt. Insbesondere eignet sich das erfindungsgemäße System auch zur Erstellung von Behandlungsplänen aller Art im medizinischen Kontext auch außerhalb von Disease management services.

## Patentansprüche

1. System zur Erstellung von Behandlungsplänen, insbesondere im Zuge von Disease management services zur Patientenaufklärung, -weiterbildung und -motivation, gegebenenfalls kombiniert mit Telemonitoring kritischer Körperwerte und daraus abgeleiteter frühzeitiger Erkennung von Risikosituationen, **gekennzeichnet durch** eine Datenbank mit einer Vielzahl von in digitaler Form gespeicherten Behandlungsmodulen, die mithilfe von Operatoren miteinander zu einem Behandlungsplan verknüpfbar sind.

2. System nach Anspruch 1, **gekennzeichnet durch** eine graphische Benutzeroberfläche zur Unterstützung der Auswahl und der Zusammenstellung von vorgegebenen Behandlungsmodulen.

3. System nach Anspruch 1 oder 2, **gekennzeichnet durch** Eingabeeinrichtungen für Patientendaten oder eine Schnittstelle zu einer elektronischen Patientenakte (EPR) zur Individualisierung des Behandlungsplans.

4. System nach einem der Ansprüche 1 bis 3, **dadurch gekennzeichnet, dass** die Behandlungsplan in maschinenlesbarer Form ausgeführt ist und automatisch Aufgaben ausführt, wie Terminverwaltung, Protokollführung, Freischalten von Informationen aus dem Internet, Versenden von Emails, Konzipieren von Formularen für Datenbanken, Verwaltung von Patientendaten od. dgl.

5. System nach einem der Ansprüche 1 bis 4, **dadurch gekennzeichnet, dass** eine weitere Datenbank zusätzlich individuell abänderbare Standard-Behandlungspläne aus einer Mehrzahl von Behandlungsmodulen für bestimmte Standardkrankheiten enthält.

6. System nach einem der Ansprüche 1 bis 5, **dadurch gekennzeichnet, dass** der Workflow der Modulkette linear ist.

7. System nach einem der Ansprüche 1 bis 6, **dadurch gekennzeichnet, dass** der Workflow der Modulkette als Entscheidungsbaum mit Entscheidungsknoten und/oder Schleifen ausgebildet ist.

8. System nach einem der Ansprüche 1 bis 7, **gekennzeichnet durch** Schaltflächen und Toolbox-Symbolleisten zur Ermöglichung eines intuitiven Arbeitens.

9. System nach einem der Ansprüche 1 bis 8, **dadurch gekennzeichnet, dass** die gewünschten Module in einer textuellen Liste oder einer Anordnung graphischer Elemente (Ikons oder dergleichen) markiert und durch bestätigenden Mausklick oder Tastendruck in die Prozesskette übernommen werden.

10. System nach einem der Ansprüche 1 bis 9, **dadurch gekennzeichnet, dass** die Datenbank (1) auf eine Datenbank (2) über das allgemeine medizinische Wissen, sowie Leitlinien und Standards und/oder auf eine Datenbank (3) über das unternehmensspezifische Wissen, wie Prozesswissen oder das patienten- und kostenträgerbezogene zielgruppenspezifische Wissen zurückgreifen kann.
